# EUROPEAN PATENT APPLICATION

(11) **EP 3 050 501 A1**
(43) Date of publication of application: **03.08.2016**
(21) Application number: 14848590.7
(22) Date of filing: 03.09.2014
(51) Int. Cl.: A61B 5/0428

(54) **CIRCUIT FOR MEASURING BIOELECTRIC SIGNAL**

(30) Priority: 25.09.2013 JP 2013197724; 25.09.2013 JP 2013197726; 25.09.2013 JP 2013197728; 25.09.2013 JP 2013197723
(71) Applicant: NISSAN MOTOR CO., LTD., Yokohama-shi, Kanagawa 221-0023 (JP); Tokyo Denki University, Tokyo 120-8551 (JP)
(72) Inventor: FUKUYAMA, Yasuhiro, Atsugi-shi Kanagawa 243-0123 (JP); SUNDA, Takashi, Atsugi-shi Kanagawa 243-0123 (JP); SHIMIZU, Youji, Atsugi-shi Kanagawa 243-0123 (JP); UENO, Akinori, Tokyo 120-8551 (JP)
(74) Representative: Grünecker Patent- und Rechtsanwälte PartG mbB
(86) International application number: PCT/JP2014/073198
(87) International publication number: WO 2015/045763

(57) **Abstract**

[SOLUTION] A reference signal is mixed with a bioelectric signal, the capacitance between a biological composition and an input means is detected from the intensity of the reference signal, and the gain of the bioelectric signal is corrected on the basis of the detected capacitance.

## Description

### Technological Field

The present invention relates to a bioelectric signal measuring circuit for measuring bioelectric signals such as an electrocardiogram or brain waves through capacitance.

### Background Art

The technique described in Patent Document 1 below has been disclosed as one type of this technique. In the document, a technique that infers a capacitance between a measuring electrode and a subject based on the area of the contact portion between the measuring electrode and the subject, and the pressure applied to the contact portion.

### Prior Art Documents

### Patent Documents

Patent Document 1: Japanese Laid-Open Patent Application No. 2009-219544

### Summary of the Invention

### Problems to be Solved by the Invention

The capacitance of the clothes worn by an occupant (subject) changes depending on factors such as the material, volume, and external pressure. However, with the technique disclosed in Patent Document 1, changes in the capacitance due to the material, volume, and the like of the clothes worn by the occupant cannot be detected. Since the gain of a bioelectric signal changes according to the capacitance, if the capacitance cannot be detected correctly, there is the risk that gain correction will not be carried out accurately.

In view of the problem described above, an object of the present invention is to provide a bioelectric signal measuring circuit that can carry out a gain correction of a bioelectric signal based on the detected capacitance, by accurately detecting the capacitance between a biological composition and an input means for inputting bioelectric signals emitted by the biological composition.

### Means Used to Solve the Problems

In order to solve the above problem, in the present invention, a reference signal is mixed with a bioelectric signal, the capacitance between a biological composition and an input means is detected from the intensity of the reference signal, and the gain of the bioelectric signal is corrected on the basis of the detected capacitance.

### Effect of the Invention

Therefore, gain correction can be accurately carried out.

### Brief Description of the Drawings

[Figure 1] Control block view of a bioelectric signal measuring circuit of the first embodiment.
[Figure 2] Schematic view of a vehicle seat of the first embodiment.
[Figure 3] Circuit diagram of an impedance conversion unit, a reference signal mixing unit, and a signal feedback unit of the first embodiment.
[Figure 4] Flowchart illustrating the flow of the steps of a capacitance measuring unit and a gain correction value calculation unit of the first embodiment.
[Figure 5] View illustrating an example of how to obtain the intensity of a reference signal of the first embodiment.
[Figure 6] Map illustrating the capacitance of the clothes in relation to the signal intensity of a reference signal of the first embodiment.
[Figure 7] Map illustrating the output gain of an impedance conversion unit in relation to the capacitance of the clothes of the first embodiment.
[Figure 8] View illustrating the relationship between the frequency and the output gain of the impedance conversion unit of when a gain correction is not carried out in the first embodiment.
[Figure 9] View illustrating the relationship between the frequency and the output gain of an impedance conversion unit of when a gain correction is carried out in the first embodiment.
[Figure 10] Control block view of a bioelectric signal measuring circuit of the second embodiment.
[Figure 11] Circuit diagram of an impedance conversion unit, a reference signal mixing unit, a signal feedback unit, and a reference signal intensity changing unit of the second embodiment.
[Figure 12] Flowchart illustrating the flow of the steps of a reference signal intensity setting value calculation unit, a capacitance measuring unit and a gain correction value calculation unit of the second embodiment.
[Figure 13] Map illustrating the capacitance of the clothes in relation to the signal intensity of a reference signal of the second embodiment.
[Figure 14] Circuit diagram of an impedance conversion unit, a reference signal mixing unit, and a signal feedback unit of the third embodiment.
[Figure 15] View illustrating the gain characteristic of the reference signal outputted from an impedance conversion unit in relation to the frequency of the reference signal, when a flattening function unit of the third embodiment is not provided.
[Figure 16] View illustrating the gain characteristic of the reference signal outputted from an impedance conversion unit in relation to the frequency of the reference signal, when a flattening function unit of the third embodiment is provided.
[Figure 17] Circuit diagram of an impedance conversion unit, a signal feedback circuit, a resonance suppressing circuit, a reference AC signal intensity analysis unit, and a reference AC signal supply circuit of the fourth embodiment.
[Figure 18] Graph representing the frequency gain characteristic of the signal outputted from an impedance conversion unit in the fourth embodiment.
[Figure 19] Graph representing the frequency gain characteristic of the signal outputted from an impedance conversion unit in the fourth embodiment.
[Figure 20] Control block view of a bioelectric signal measuring circuit of the fifth embodiment.
[Figure 21] Flowchart illustrating the flow of the steps of a capacitance measuring unit, a gain correction value calculation unit, and a reference signal generating unit of the fifth embodiment.
[Figure 22] Map illustrating the reference signal in relation to the capacitance of the clothes of the fifth embodiment.
[Figure 23] Control block view of a bioelectric signal measuring circuit of the sixth embodiment.
[Figure 24] Flowchart illustrating the flow of the steps of a signal selection unit of the sixth embodiment.
[Figure 25] Control block view of a bioelectric signal measuring circuit of the seventh embodiment.
[Figure 26] Control block view of a bioelectric signal measuring circuit of the eighth embodiment.
[Figure 27] Control block view of a bioelectric signal measuring circuit of the ninth embodiment.
[Figure 28] Schematic view of a vehicle seat of the ninth embodiment.
[Figure 29] Graph illustrating the frequency output gain characteristic per capacitance of the clothes of the ninth embodiment.
[Figure 30] View illustrating an example of a limb lead of the eleventh embodiment.
[Figure 31] Graph illustrating an example of the changes in the capacitance between an electrode and a biological composition of the thirteenth embodiment.
[Figure 32] Control block view of a bioelectric signal measuring circuit of the fifteenth embodiment.

### Reference Signs List

- 1: circuit for measuring bioelectric signal
- 2: electrode (input means)
- 3: seat
- 6: impedance conversion unit
- 7: reference signal mixing unit
- 7a: flattening function unit
- 8: signal feedback unit
- 9: signal separating unit
- 10: capacitance measuring unit
- 11: gain correction value calculation unit
- 12: bioelectric signal gain correction unit
- 13: reference signal intensity setting value calculation unit
- 14: reference signal intensity changing unit
- 16: reference signal calculation unit (artifact calculating means)
- 18: subtraction unit (artifact removing means)
- 19: signal selection unit
- 20: reliability information collecting unit
- 24: bioelectric signal measuring unit (bioelectricity measuring means)
- 25: external information source (behavior inferring means)
- 27: correlation evaluating unit

### Preferred Embodiments of the Invention

### [Embodiment 1]

### [Circuit for measuring bioelectric signal]

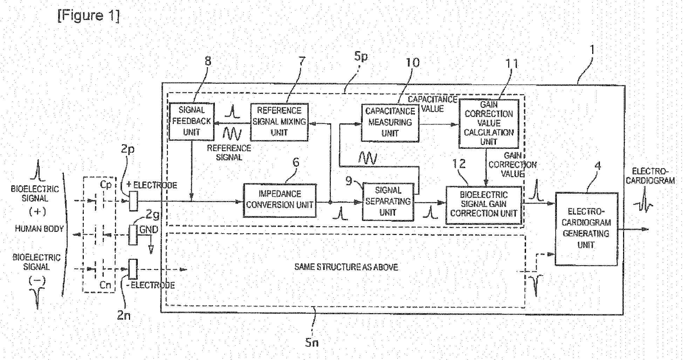

Figure 1 is a control block view of a bioelectric signal measuring circuit 1. The bioelectric signal measuring circuit 1 inputs a bioelectric signal of a human body from a positive electrode 2p and a negative electrode 2n, and outputs the same as an electrocardiogram.

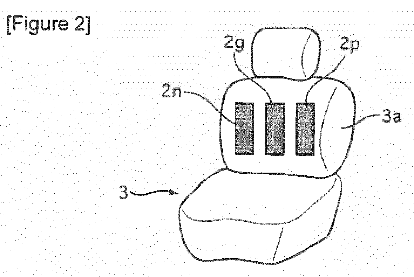

Figure 2 is a schematic view of a seat 3 of a vehicle. A positive electrode 2p and a negative electrode 2n are disposed, separated to the left and right on the surface of a seat back 3a, having insulating properties, of the seat 3. Additionally, a ground 2g is disposed between the positive electrode 2p and the negative electrode 2n. A bioelectric signal can thereby be measured simply by an occupant sitting in the seat 3. The electrodes 2 are made of materials having conductive properties, such as metal materials including gold, silver, copper or nichrome, carbon-based materials such as carbon or graphite, particulate materials made of semiconductors of metals and metal oxides, conductive polymer materials such as acetylene-based, 5-membered heterocyclic, phenylene-based or aniline-based compounds, or the like.

The bioelectric signal measuring circuit 1 comprises a gain correction unit 5 for correcting the gain in response to the capacitance of the clothes worn by the occupant, and an electrocardiogram generating unit 4 for generating an electrocardiogram from the signal after gain correction. The gain correction unit 5 is configured from a positive side gain correction unit 5p for correcting the gain of the bioelectric signal inputted from the positive electrode 2p, and a negative side gain correction unit 5n for correcting the gain of the bioelectric signal inputted from the negative electrode 2n. However, since the configurations of the positive side gain correction unit 5p and the negative side gain correction unit 5n are identical, each configuration will be described below as a gain correction unit 5 without distinguishing between the positive side gain correction unit 5p and the negative side gain correction unit 5n.

A gain correction unit 5 comprises an impedance conversion unit 6, a reference signal mixing unit 7, a signal feedback unit 8, a signal separating unit 9, a capacitance measuring unit 10, a gain correction value calculation unit 11, and a bioelectric signal gain correction unit 12.

### <Impedance conversion unit>

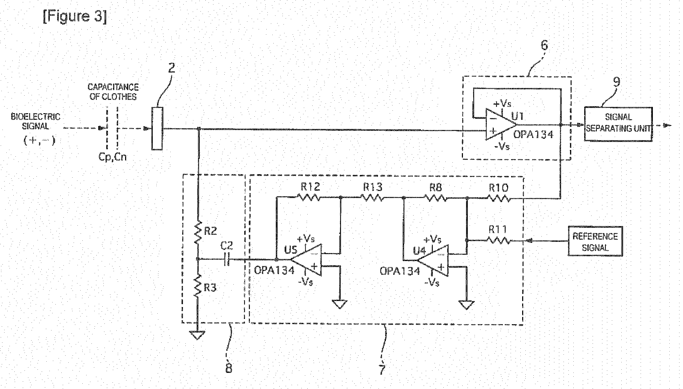

Figure 3 is a circuit diagram of the impedance conversion unit 6, the reference signal mixing unit 7, and the signal feedback unit 8.

The impedance conversion unit 6 detects a bioelectric signal inputted to the electrode 2. The impedance conversion unit 6 is configured from a voltage follower circuit based on an operational amplifier, as illustrated in Figure 3.

### <Reference signal mixing unit>

The reference signal mixing unit 7 mixes and outputs the output of the impedance conversion unit 6 and the reference signal for measuring the capacitance of the clothes worn by the occupant.

An AC signal is used for the reference signal. If a DC signal is used as a reference signal, since the offset electric current of the voltage follower circuit of the impedance conversion unit 6 will be affected, there is the risk that in some cases the output electric current of the voltage follower circuit will be saturated. The frequency of the reference signal is preferably set avoiding the R wave signal frequency band 10-40 Hz in order to avoid interference with the R wave signal of the electrocardiogram.

A frequency signal fixed by an oscillating circuit such as a solid vibrator oscillating circuit, a CR oscillating circuit or an LC oscillating circuit can be used for the reference signal, and waveforms programmed to a microcomputer can be outputted by means of a D/A converter and used.

The reference signal mixing unit 7 is configured from two inverting adder circuits being two-stage connected, as illustrated in Figure 3. The output of the impedance conversion unit 6 and the reference signal can thereby be mixed.

### <Signal feedback unit>

A signal feedback unit 8 is connected to the output side of the reference signal mixing unit 7. The signal feedback unit 8 is configured from a bootstrap circuit, as illustrated in Figure 3. The output of the signal feedback unit 8 is fed back to the input side of the impedance conversion unit 6.

### <Signal separating unit>

A signal separating unit 9 is configured from a bandpass filter circuit for extracting the frequency band of the R wave 10-40 [Hz] of an electrocardiogram, which is a bioelectric signal, and a bandpass filter circuit for extracting the frequency band other than the R wave. Accordingly, separating the bioelectric signal and the reference signal is possible.

### <Capacitance measuring unit and Gain correction value calculation unit>

A capacitance measuring unit 10 and a gain correction value calculation unit 11 are software installed to a microprocessor having an A/D converter.

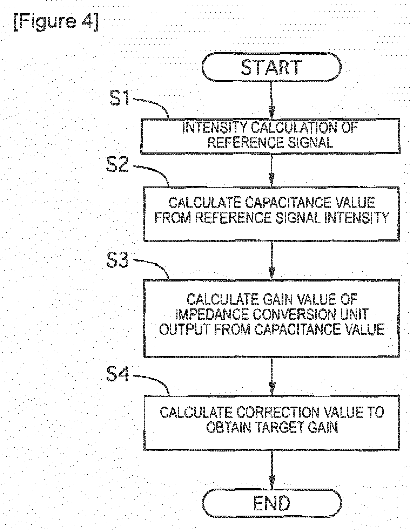

Figure 4 is a flowchart illustrating the flow of the steps. Steps S1 and S2 are steps of the capacitance measuring unit 10, and steps S3 and S4 are steps of the gain correction value calculation unit 11.

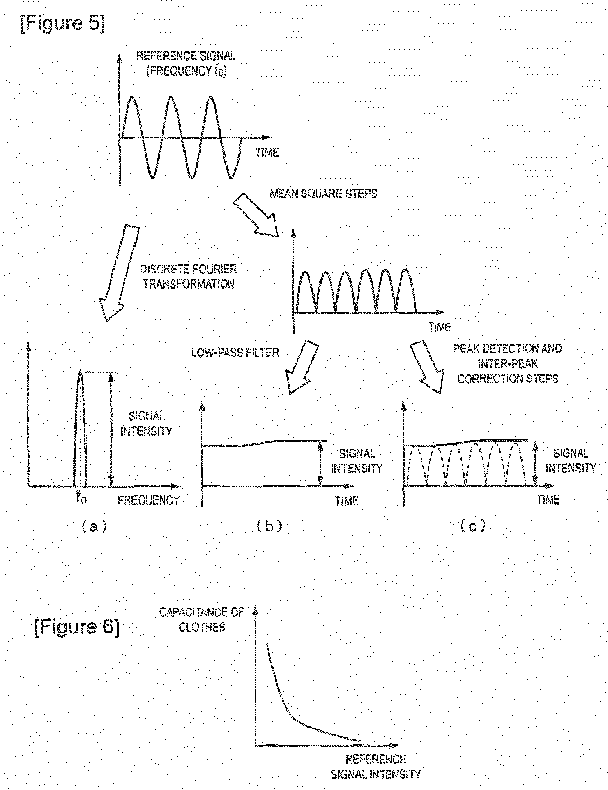

In step S1, the signal intensity of the reference signal is calculated. Figure 5 is a view illustrating an example of how to obtain the intensity of a reference signal. Here, three examples of how to obtain the signal intensity of a reference signal will be shown. In the first example, signal strength is obtained by carrying out a discrete Fourier transformation to the reference signal (Figure 5(a)). In the second example, signal strength is obtained in chronological order by carrying out a low-pass filter step to the mean square of the reference signal (Figure 5(b)). In the third example, signal strength is obtained in chronological order by carrying out an inter-peak correction step to the mean square of the reference signal (Figure 5(c)).

In step S2, the capacitance of the clothes is calculated from the signal intensity of the reference signal. Figure 6 is a map illustrating the capacitance of the clothes in relation to the signal intensity of a reference signal. In step S2, the capacitance of the clothes is calculated using the map in Figure 6.

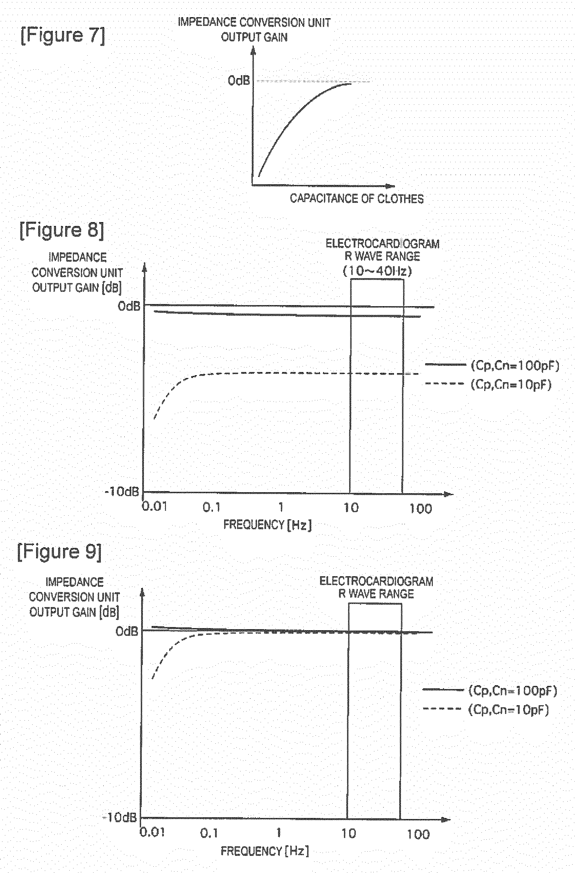

In step S3, the output gain of the impedance conversion unit 6 is calculated from the calculated capacitance. Figure 7 is a map illustrating the output gain of an impedance conversion unit 6 in relation to the capacitance of the clothes. In step S3, the output gain of the impedance conversion unit 6 is calculated using the map in Figure 7.

In step S4, the gain correction value is calculated in accordance with the calculated output gain.

Above, the signal intensity calculation of the reference signal in step S1 is also implemented as software, but the same may be configured from an analogue circuit capable of carrying out a similar step using, for instance, a wave detecting circuit.

### <Bioelectric signal gain correction unit>

A bioelectric signal gain correction unit 12 carries out a correction of the bioelectric signal gain inputted from the signal separating unit 9 with the gain correction value inputted from the gain correction value calculation unit 11. The bioelectric signal gain correction unit 12 is configured by combining an amplifier circuit, an attenuation circuit, and the like, capable of setting the inputted signal to any magnification.

### <Electrocardiogram generating unit>

Bioelectric signal after gain correction is inputted from the gain correction unit 5 to the electrocardiogram generating unit 4. A filtering step and an amplification step are carried out to each of the positive side and negative side bioelectric signals, and an electrocardiogram is obtained by taking the difference between the positive side signal and the negative side signal after the steps.

### [Effects]

Clothes having a capacitance (Cp, Cn) is interposed between the electrode 2 and the human body, since the occupant sits in the seat 3 wearing clothes. The capacitance of the clothes changes depending on factors such as the material and volume of the clothes, or external pressure. Since the gain of a bioelectric signal changes according to the capacitance, if the capacitance cannot be detected correctly, the gain correction will not be carried out accurately. Additionally, if the positive side capacitance Cp and the negative side capacitance Cn are different, the signal after taking the difference between the positive side signal and the negative side signal after each of the steps at the electrocardiogram generating unit 4 will include error.

Therefore, in the first embodiment, the output of the impedance conversion unit 6 and the reference signal for measuring the capacitance of the clothes are mixed, and the mixed signal is returned to the impedance conversion unit 6. That is, the occupant side also becomes a ground, and the reference signal also flows to the occupant side. Therefore, the capacitance of the clothes becomes a voltage dividing factor of the reference signal, and the signal intensity of the reference signal will change in response to the capacitance. The capacitance of the clothes can be obtained from the signal intensity of the reference signal, by separating the reference signal from the signal outputted from the impedance conversion unit 6. Then, the changing of the bioelectric signal gain due to a change in the capacitance of the clothes can be suppressed, by the gain being corrected to a predetermined value set in advance.

The following relationship exists between the capacitance of the clothes and the output gain of the impedance conversion unit 6 as the capacitance of the clothes increases, the gain becomes closer to 0 [dB]. Figure 8 is a view illustrating the relationship between the frequency and the output gain of the impedance conversion unit 6 of when a gain correction is not carried out. The gain in response to the frequency of when the capacitance (Cp, Cn) of the clothes is 110 [pF] (shown by the solid line) and 10 [pF] (shown by the broken line) are illustrated in Figure 8. According to Figure 8, in the R wave region (10-40 [Hz]) of the electrocardiogram, when the capacitances are 110 [pF] and 10 [pF], the gains are -0.4 [mdB] and -3.5 [dB], respectively; therefore, a difference in the gain is generated, caused by the difference in the capacitances.

By incorporating the above relationship in a software program as a database or a calculation formula on a microprocessor in advance, the difference in the gain due to the capacitance can be eliminated. For example, if the target value of the output gain of the gain correction unit 5 is to be 0 [dB], corrections may be carried out so that when the capacitances of the clothes are 110 [pF] and 10 [pF], the gains become +0.4 [mdB] and +3.5 [dB], respectively. The target value of the output gain of the gain correction unit 5 is not limited to 0 [dB] and may be set arbitrarily.

Figure 9 is a view illustrating the relationship between the frequency and the output gain of the impedance conversion unit 6 of when a gain correction is carried out. The gain in response to the frequency of when the capacitance (Cp, Cn) of the clothes is 110 [pF] (shown by the solid line) and 10 [pF] (shown by the broken line) are illustrated in Figure 9. According to Figure 9, after the gain correction, in the R wave region (10-40 [Hz]) of the electrocardiogram, when the capacitances are 110 [pF] and 10 [pF], the gains are both 0 [dB]; therefore, a difference in the gain due to the difference in the capacitances is eliminated.

Additionally, an electrode 2 is installed in the seat back 3a of the seat 3 of a vehicle. Therefore, a bioelectric signal can be measured as long as the occupant is seated in the seat 3. For example, if the electrode 2 is installed in a steering wheel, etc., a bioelectric signal cannot be measured once the occupant releases the steering wheel. While in the vehicle, the occupant is basically seated in the seat 3; therefore, bioelectric signal of the occupant while in the vehicle can be constantly measured in the first embodiment.

### [Effects]

(1) The invention is configured to comprise:
   an electrode 2 (input means) for inputting bioelectric signals emitted by an occupant (biological composition);
   an impedance conversion unit 6 which carries out an impedance conversion of the bioelectric signal inputted to the electrode 2;
   a reference signal mixing unit 7 which mixes the output signal of the impedance conversion unit 6 and the reference signal for measuring the capacitance between the occupant and the electrode 2 (i.e., clothes);
   a signal feedback unit 8 for feeding back the output signal of the reference signal mixing unit 7 to the impedance conversion unit 6;
   a signal separating unit 9 for separating the bioelectric signal and the reference signal from the output signal of the impedance conversion unit 6;
   a capacitance measuring unit 10 for calculating the capacitance from the signal intensity of the reference signal inputted from the signal separating unit 9;
   a gain correction value calculation unit 11 for calculating the gain correction value of the bioelectric signal based on the capacitance calculated by the capacitance measuring unit 10; and
   a bioelectric signal gain correction unit 12 for carrying out the gain correction of the bioelectric signal based on the gain correction value.
   Therefore, the capacitance of the clothes worn by the occupant can be accurately determined, and the bioelectric signal gain which changes in response to the capacitance can be corrected to a predetermined value. Therefore, an accurate electrocardiogram can be obtained from the bioelectric signal.
(2) The electrode 2 is configured to be installed in the seat 3 of the vehicle.
   Therefore, the bioelectric signal of the occupant while in the vehicle can constantly be measured.

### [Embodiment 2]

### [Circuit for measuring bioelectric signal]

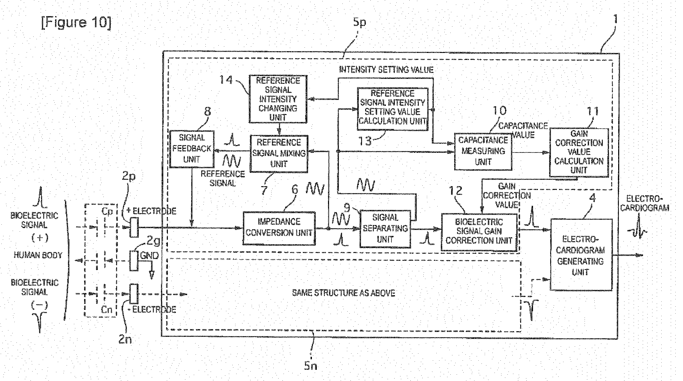

Figure 10 is a control block view of a bioelectric signal measuring circuit 1. The bioelectric signal measuring circuit 1 of the second embodiment differs from the bioelectric signal measuring circuit 1 of the first embodiment in that a reference signal intensity setting value calculation unit 13 and a reference signal intensity changing unit 14 are provided, as well as in the content of the steps of the capacitance measuring unit 10. In the second embodiment, the configurations that differ from the first embodiment will be mainly explained, while the configurations that are the same as the first embodiment are given the same codes, and the descriptions thereof are omitted.

### <Reference signal intensity changing unit>

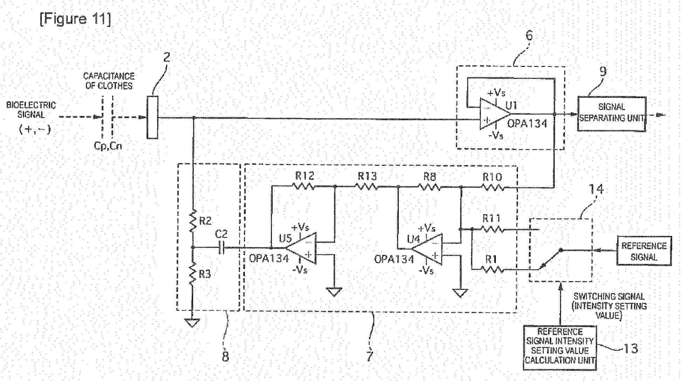

Figure 11 is a circuit diagram of an impedance conversion unit 6, a reference signal mixing unit 7, a signal feedback unit 8, and a reference signal intensity changing unit 14 of the second embodiment. The impedance conversion unit 6, the reference signal mixing unit 7, and the signal feedback circuit 8 are substantially the same as the circuit illustrated in Figure 3 of the first embodiment. However, two terminals for inputting the reference signal of the reference signal mixing unit 7 are provided, and each terminal has a different resistance. That is, the resistance R11 of one terminal side is 10 [kΩ], and the resistance R1 of the other terminal side is I [kΩ].

The reference signal intensity changing unit 14 is a switch circuit for selecting which input terminal of the reference signal mixing unit 7 to input the reference signal to. Selection of the input terminals is determined according to the intensity setting value obtained in the reference signal intensity setting value calculation unit 13, which will be described below.

### <Reference signal intensity setting value calculation unit and capacitance measuring unit>

The reference signal intensity setting value calculation unit 13 inputs a reference signal from the signal separating unit 9, and, according to the signal intensity of the inputted reference signal, sets the signal intensity of the reference signal to be inputted to the reference signal mixing unit 7 (intensity setting value).

The capacitance measuring unit 10 calculates the capacitance of the clothes according to the signal intensity of the reference signal, using a map corresponding to the intensity setting value.

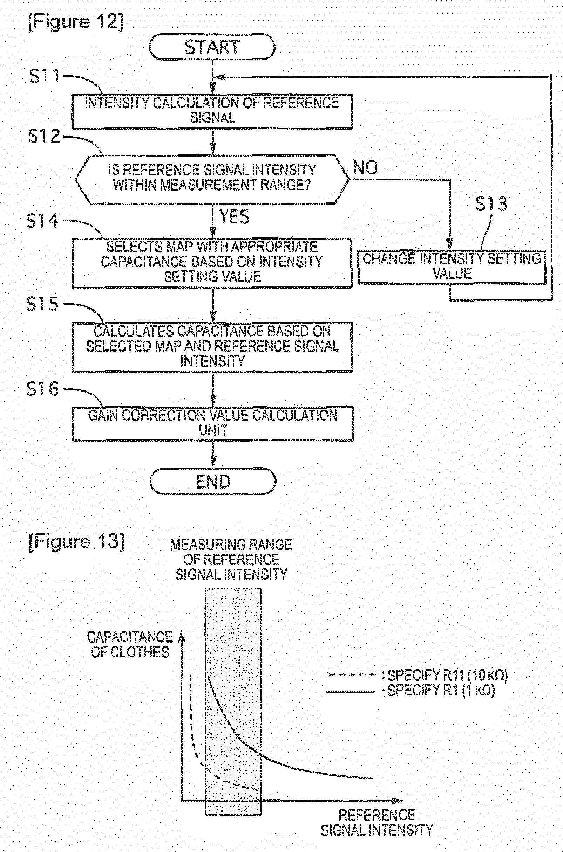

Figure 12 is a flowchart illustrating the flow of the steps. Steps S 11, S12, and S13 are steps of the reference signal intensity setting value calculation unit 13, steps S 14 and S15 are steps of the capacitance measuring unit 10, and step 16 is a step of the gain correction value calculation unit 11. The step of the gain correction value calculation unit 11 in step S16 carries out the same step as the steps of the first embodiment (Steps S3 and S4 in Figure 4). The reference signal intensity setting value calculation unit 13, the capacitance measuring unit 10, and the gain correction value calculation unit 11 are software installed in a microprocessor having an A/D converter.

In step S11, the signal intensity of the reference signal is calculated. The method of calculating the signal intensity is by carrying out the same steps as step S1 in Figure 4 of the first embodiment.

In step S12, whether or not the calculated signal intensity of the reference signal is within the measurement range of the capacitance measuring unit 10 is determined; the steps proceeds to step S 14 when the intensity is within the measurement range, and the steps proceeds to step S13 when the intensity is outside of the measurement range.

In Step S13, the setting value (intensity setting value) of the signal intensity of the reference signal inputted to the reference signal mixing unit 7 is changed. The intensity setting value may be set in multiple steps or in a stepless manner, and is set in two steps of large and small in the second embodiment. If the intensity setting value is set large, the input terminal provided with the resistor R1 will be selected in the reference signal intensity changing unit 14, and if the intensity setting value is set small, the input terminal provided with the resistor R11 will be selected in the reference signal intensity changing unit 14.

In step S14, the capacitance of the clothes is calculated from the signal intensity of the reference signal. Figure 13 is a map illustrating the capacitance of the clothes in relation to the signal intensity of a reference signal. In the second embodiment, there are two maps in accordance with whether the intensity setting value large or small. In other words, the map shown by the dotted line in Figure 13 is selected when the intensity setting value is large and the input terminal of the resistor R11 is selected in the reference signal intensity changing unit 14. On the other hand, the map shown by the solid line in Figure 13 is selected when the intensity setting value is small and the input terminal of the resistor R1 is selected in the reference signal intensity changing unit 14.

In step S15, the capacitance of the clothes is calculated using the selected map.

In step S16, the gain correction value is calculated in the same manner as in the first embodiment.

### [Effects]

If the signal intensity of the reference signal inputted to the capacitance measuring unit 10 is low, the resolution after the A/D conversion deteriorates, and the measurement precision is degraded. On the other hand, if the signal intensity is high, the input range of the A/D converter is exceeded thereby, and the measurement of the capacitance cannot be carried out.

Therefore, in the second embodiment, whether or not the signal intensity of the reference signal inputted from the signal separating unit 9 is within a range in which the capacitance is measurable in the capacitance measuring unit 10 is determined in the reference signal intensity setting value calculation unit 13. Then, if the intensity is outside of the measurable range, the intensity setting value is changed, and the signal intensity of the reference signal inputted to the reference signal mixing unit 7 is changed.

Accordingly, the signal intensity of the reference signal inputted to the capacitance measuring unit 10 can be set adequately, and the capacitance can be measured with high precision.

Additionally, in the second embodiment, two terminals are provided for inputting the reference signal of the reference signal mixing unit 7, and a different resistor is installed to each thereof. Additionally, the reference signal intensity changing unit 14 is configured from a switch circuit for selecting to which input terminal of the reference signal mixing unit 7 the reference signal is inputted. Therefore, the signal intensity of the reference signal inputted to the reference signal mixing unit 7 can be changed with a simple configuration.

### [Effects]

(3) The invention is configured to comprise:
   a reference signal intensity setting value calculation unit 13 for setting the intensity of the reference signal inputted to the reference signal mixing unit 7 from the intensity of the reference signal inputted from the signal separating unit 9; and
   a reference signal intensity changing unit 14 for changing the intensity of the reference signal inputted to the reference signal mixing unit 7 according to the intensity of the reference signal set by the reference signal intensity setting value calculation unit 13, wherein
   the capacitance measuring unit 10 is configured to calculate the capacitance from the intensity of the reference signal inputted from the signal separating unit 9 and the intensity of the reference signal set by the reference signal intensity setting value calculation unit 13.
   Therefore, the signal intensity of the reference signal inputted to the capacitance measuring unit 10 can be set adequately, and the capacitance can be measured with high precision.
(4) The reference signal intensity changing unit 14 is configured so that the changing of the intensity of the reference signal inputted to the reference signal mixing unit 7 is carried out by changing a constant of the electric circuit in the reference signal mixing unit 7.

Therefore, the signal intensity of the reference signal inputted to the reference signal mixing unit 7 can be changed with a simple configuration.

### [Embodiment 3]

### [Bioelectric signal measuring circuit]

The bioelectric signal measuring circuit 1 of the third embodiment partially differs from the bioelectric signal measuring circuit 1 of the first embodiment in the configuration of the reference signal mixing unit 7. In the third embodiment, the configurations that differ from the first embodiment will be mainly explained, while the configurations that are the same as the first embodiment are given the same codes, and the descriptions thereof are omitted.

### <Reference signal mixing unit>

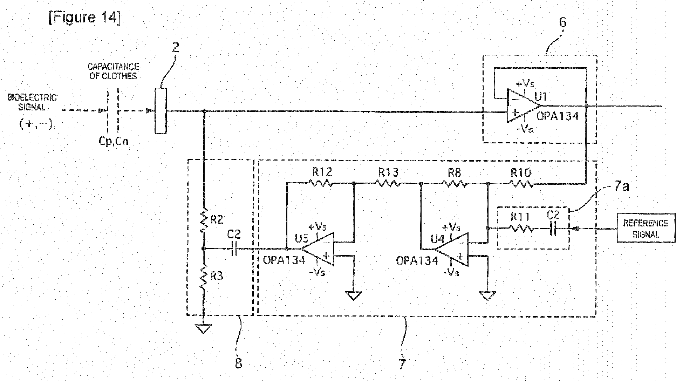

Figure 14 is a circuit diagram of the impedance conversion unit 6, the reference signal mixing unit 7, and the signal feedback unit 8. The reference signal mixing unit 7 is provided with a flattening function unit 7a in the input section for the reference signal. The flattening function unit 7a is an RC series circuit, in which a resistor R11 and a capacitor C1 are connected in series, as illustrated in Figure 14.

### [Effects]

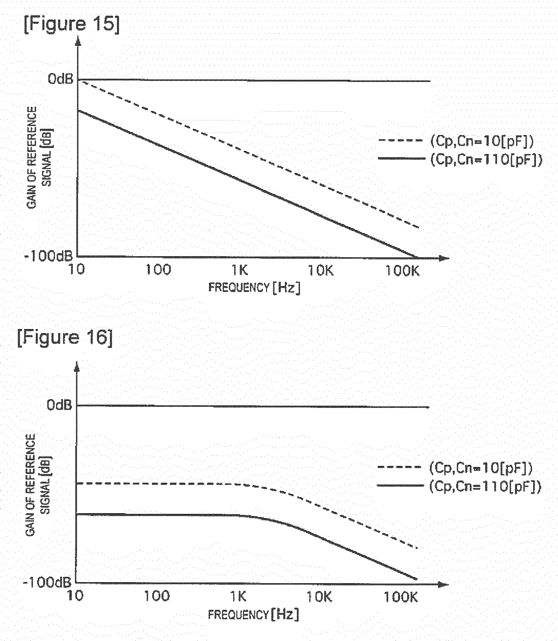

Figure 15 is a view illustrating the gain characteristic of the reference signal outputted from an impedance conversion unit 6 in relation to the frequency of the reference signal of when a flattening function unit 7a is not provided. When the frequency of a reference signal has deviated from a desired value due to a variation in the temperature characteristic and the individual characteristic, a deviation also occurs in the gain of the reference signal outputted from the impedance conversion unit 6, as illustrate in Figure 15.

Therefore, in the third embodiment, the change of the gain corresponding to the frequency change of the reference signal is flattened, by providing a flattening function unit 7a. Figure 16 is a view illustrating the gain characteristic of the reference signal outputted from an impedance conversion unit 6 in relation to the frequency of the reference signal of the third embodiment of when a flattening function unit 7a is provided. The gain characteristic of the reference signal is flattened in the frequency band of about 10 [Hz] to about 1 [kHz], and even if the frequency of the reference signal deviates more or less, changes in the gain will be small, as illustrated in Figure 16. Therefore, the capacitance can be measured with high precision.

### [Effects]

(5) The reference signal mixing unit 7 is configured to comprise a flattening function unit 7a set to flatten the gain characteristic of the reference signal outputted from the impedance conversion unit 6 in relation to the frequency change of the reference signal.

Therefore, since the gain characteristic of the reference signal is flattened in relation to the frequency change of the reference signal, even if the frequency of the reference signal deviates more or less from the desired value, changes in the gain will be small, and the capacitance can be measured with high precision.

### [Embodiment 4]

### [Circuit for measuring bioelectric signal]

The bioelectric signal measuring circuit 1 of the fourth embodiment partially differs from the bioelectric signal measuring circuit 1 of the first embodiment in the configuration of the signal feedback unit 8. Additionally, in the fourth embodiment, a resonance suppressing unit 15 is provided. In the fourth embodiment, the configurations that differ from the first embodiment will be mainly explained, while the configurations that are the same as the first embodiment are given the same codes, and the descriptions thereof are omitted.

### <Signal feedback unit>

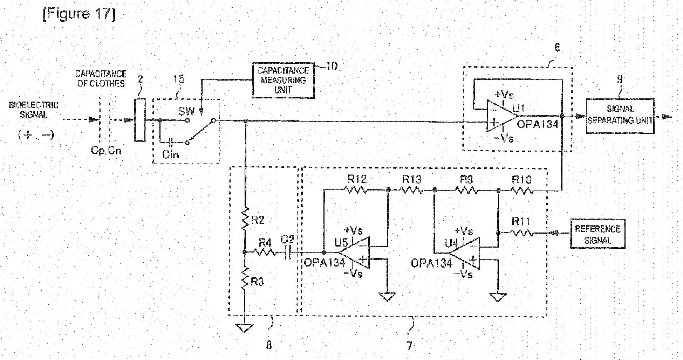

Figure 17 is a circuit diagram of the impedance conversion unit 6, the reference signal mixing unit 7, the signal feedback unit 8, and the resonance suppressing unit 15.

The signal feedback unit 8 is configured from two resistors R2 and R3 connected in series in a circuit connected to the input terminal of the impedance conversion unit 6 and a ground, a resistor R4, and a capacitor C2 directly connected in a circuit connecting the resistors R2 and R3 and the output terminal of the impedance conversion unit 6. In other words, the resistor R4 is added to the configuration of the signal feedback unit 8 of the first embodiment.

### <Resonance suppressing circuit>

The resonance suppressing unit 15 is configured from a capacitor Cin and a switch SW. The switch SW switches between a circuit that passes through the capacitor Cin, and a circuit that does not pass through the capacitor Cin, in accordance with the reference signal intensity calculated by the capacitance measuring unit 10. The circuit that passes through the capacitor Cin is selected when the capacitance of the clothes worn by the occupant is determined to be large by the reference signal intensity. On the other hand, when the capacitance of the clothes is determined to be small, the circuit that does not pass through the capacitor Cin is selected.

### [Effects]

An electrical circuit must be configured to use large lead wire resistors and capacitors in order to carry out a stable measuring of the bioelectric signal. On the other hand, it is conceivable to use chip-type components in place of the lead wire resistors and capacitors in order to reduce the size and cost of the apparatus. However, with chip-type components, the resistance value, capacitance, and the like, are smaller than that of lead wire type components, due to the restrictions of withstanding voltage characteristics and dimensions. Therefore, a resonance point is generated in the frequency gain characteristic, and particularly when measuring a bioelectric signal in environments where vibration occurs, there is the problem that the measurement becomes unstable.

Therefore in the fourth embodiment, the signal feedback unit 8 is configured from two resistors R2 and R3 connected in series in a circuit connected to the input terminal of the impedance conversion unit 6 and a ground, a resistor R4 and a capacitor C1 connected in series in a circuit connecting between the two resistors R2 and R3 and the output terminal of the impedance conversion unit 6.

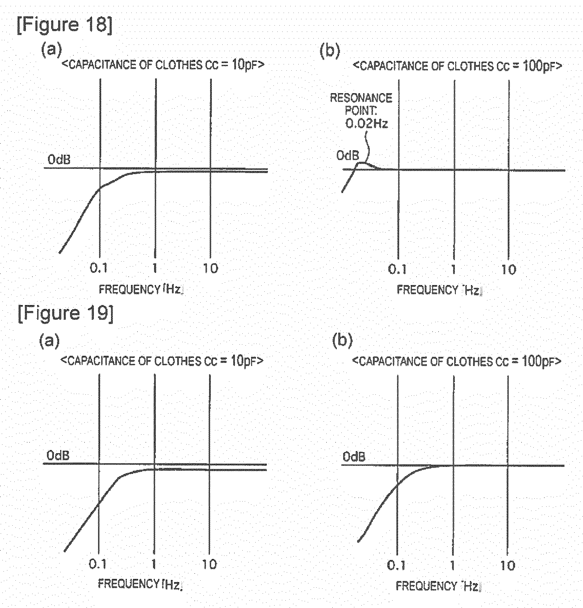

Figure 18 is a graph representing the frequency gain characteristic of the signal outputted from the impedance conversion unit 6 of when the capacitance Cc of the clothes is 10 [pF] (Figure 18 (a)) and 100 [pF] (Figure 18 (b)). Figure 18 illustrates the characteristic of when the resistance value of the resistors R2 and R3 are both 51 [MΩ], the resistance value of the resistor R4 is 20 [kΩ], and the capacitance of the capacitor C2 is 470 [pF]; the values are set to values usable as chip components. Figure 18 illustrates a state in which a circuit that does not pass through the capacitor Cin is selected in the resonance suppressing unit 15.

When the capacitance Cc is 10 [pF], a frequency gain characteristic free of resonance is obtained, as illustrated in Figure 18 (a), and measuring of a bioelectric signal can be stably carried out even in a vibrating environment. On the other hand, when the capacitance Cc is 100 [pF], although a resonance is occurring in the vicinity of 0.02 [Hz], as illustrated in Figure 18(b), a stable frequency gain characteristic is obtained in the R wave region (10-40 [Hz]) of the electrocardiogram, and measuring of a bioelectric signal can be stably carried out even in a vibrating environment.

However, as described above, when the capacitance Cc is 100 [pF], a resonance is occurring in the vicinity of 0.02 [Hz], as illustrated in Figure 18(b). Therefore, in the fourth embodiment, resonance is configured to be suppressed when the capacitance Cc of the clothes is relatively high (100 [pF]), further by the capacitor Cin of the resonance suppressing unit 15.

Figure 19 is a graph representing the frequency gain characteristic of the signal outputted from the impedance conversion unit 6 of when the capacitance Cc of the clothes is 10 [pF] (Figure 19(a)) and 100 [pF] (Figure 19(b)), Figure 19 illustrates the characteristic of when the resistance values of the resistors R2 and R3 are both 51 [MΩ], the resistance value of the resistor R4 is 20 [kΩ], the capacitance of the capacitor C1 is 470 [pF]; and the capacitance of the capacitor Cin is 10 [pF]; the values are set to values usable as chip components. Figure 18 illustrates a state in which a circuit that does pass through the capacitor Cin is selected in the resonance suppressing unit 15.

When the capacitance Cc is 10 [pF], a gain characteristic free of resonance is obtained, as illustrated in Figure 19(a), and measuring of a bioelectric signal can be stably carried out even in a vibrating environment. However, the overall gain is decreased compared to a circuit (Figure 18(a)) that does not pass through the capacitor Cin in the resonance suppressing unit 15. On the other hand, when the capacitance Cc is 100 [pF], a gain characteristic free of resonance is obtained, as illustrated in Figure 19(b), and measuring of a bioelectric signal can be stably carried out even in a vibrating environment.

Therefore, by adding a resistor R4 connected in series to the capacitor C2 in the signal feedback unit 8, a frequency gain free of resonance can be obtained, regardless of the magnitude of the capacitance Cc of the clothes, in the R wave region (10-40 [Hz]) of an electrocardiogram. Therefore, measuring of a bioelectric signal can be stably carried out even in a vibrating environment.

Additionally, when the capacitance Cc of the clothes is relatively high (100 [pF]), a frequency gain free of resonance can be obtained by the capacitor Cin by adding the resonance suppressing unite 15. On the other hand, when the capacitance Cc of the clothes is relatively small (10 [pF]), a circuit that does not pass through the capacitor Cin is selected, and the decreasing of the gain due to the capacitor Cin can be avoided.

### [Effects]

The effects of the fourth embodiment will be described below.

(6) The signal feedback unit 8 comprises two resistors R2 and R3 connected in series in a circuit connected to the input terminal of the impedance conversion unit 6 and a ground, and a capacitor C2 and a resistor R4 connected in series in a circuit connecting between the resistors R2 and R3 and the output terminal of the reference signal mixing unit 7, wherein, when the capacitance measured by the capacitance measuring unit 10 is larger than a predetermined value, a capacitor Cin is provided, which is connected in series between an electrode 2 and the input terminal of the impedance conversion unit 6.

Therefore, a stable frequency gain characteristic can be obtained at a high value, regardless of the high/low of the capacitance of the clothes, and measuring of a bioelectric signal can be stably carried out even in a vibrating environment.

### [Embodiment 5]

### [Circuit for measuring bioelectric signal]

The bioelectric signal measuring circuit 1 of the fifth embodiment differs from the bioelectric signal measuring circuit 1 of the first embodiment in that a reference signal calculation unit 16, a filter/amplifier unit 17, and a subtraction unit 18 are provided. In the fifth embodiment, the configurations that differ from the first embodiment will be mainly explained, while the configurations that are the same as the first embodiment are given the same codes, and the descriptions thereof are omitted.

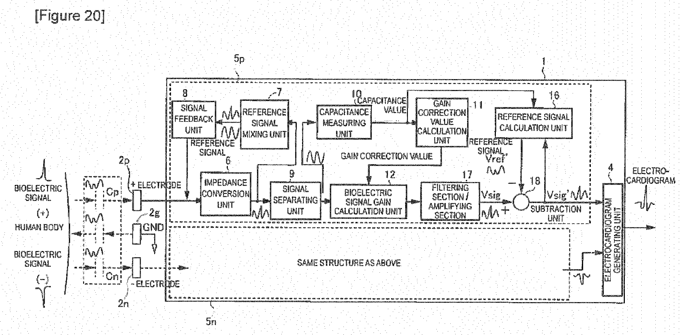

Figure 20 is a control block view of a bioelectric signal measuring circuit 1. The bioelectric signal measuring circuit I inputs a bioelectric signal of a human body from a positive electrode 2p and a negative electrode 2n, and outputs the same as an electrocardiogram.

### <Reference signal generating unit>

The reference signal calculation unit 16 is a software installed to a microprocessor having an A/D converter.

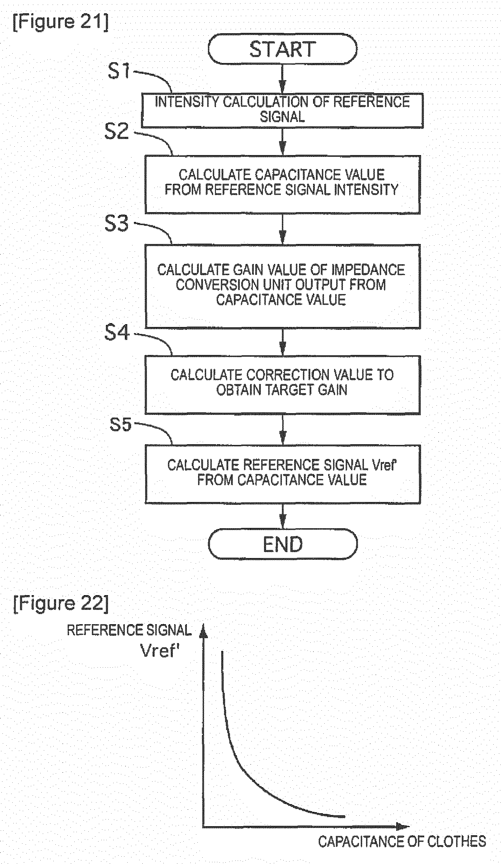

Figure 21 is a flowchart illustrating the flow of the steps. Steps S1 and S2 are steps of the capacitance measuring unit 10, steps S3 and S4 are steps of the gain correction value calculation unit 11, and step S5 is a step of the reference signal calculation unit 16.

Since the steps from step S1 to step S4 are the same as those described in the first embodiment using Figure 4, the descriptions thereof will be omitted.

In step S5, a reference signal (voltage) Vref is calculated from the calculated capacitance. Figure 22 is a map illustrating the reference signal Vref in relation to the capacitance of the clothes. In step S5, the reference signal Vref is calculated using the map of Figure 22. The reference signal Vref has a substantially inversely proportional relationship with the capacitance.

### <Filter/amplifier unit>

The filter/amplifier unit 17 respectively carries out a filtering step and an amplification step on the bioelectric signals so that the signal shall be appropriate for an electrocardiogram measurement. The filter/amplifier unit 17 can be realized as an electric circuit configured from a general-purpose analog 1C. Then, the signal is converted to a digital signal (bioelectric signal Vsig) in the A/D converter connected to a microprocessor.

### <Subtraction unit>

In the subtraction unit 18, the reference signal (voltage) Vref is subtracted from the bioelectric signal (voltage) Vsig outputted from the filter/amplifier unit 17, and the signal after the subtraction is outputted as a bioelectric signal (voltage) Vsig'. In the subtraction unit 18, the reference signal Vref is determined so that the error ΔV = RMS (Vsig - Vref) of when the bioelectric signal Vsig is subtracted using the reference signal Vref is at a minimum. α is determined by the formula: reference signal Vref = α * Vref Here, RMS represents the Root Mean Square.

### <Electrocardiogram generating unit>

The Electrocardiogram generating unit 4 inputs a bioelectric signal Vsig' from the subtraction unit 18. An electrocardiogram is obtained by taking the difference between the positive side and negative side bioelectric signals Vsig'. In the first embodiment, the filtering steps and the amplification steps are also carried out in the electrocardiogram generating unit 4. In the fifth embodiment, the filtering steps and the amplification steps are not carried out in the electrocardiogram generating unit 4, since the filtering steps and the amplification steps are carried out in the filter/amplifier unit 17.

### [Effects]

Clothes having a capacitance (Cp, Cn) is interposed between the electrode 2 and the human body, since the occupant sits in the seat 3 wearing clothes. The capacitance of the clothes changes depending on factors such as the material and volume of the clothes, or external pressure. Therefore, if the occupant moves due to a vibration of the vehicle, etc., the capacitance of the clothes changes. Since an electric charge transference occurs every time the capacitance changes, noise due to the electric charge transference occurs in the bioelectric signal. This noise will be referred to as an artifact. An artifact is the generic term for any signal, other than the bioelectric signal, which is mixed into the bioelectric signal. If an artifact is mixed in, an electrocardiogram cannot be measured accurately.

Therefore, the fifth embodiment is configured so that the artifact (reference signal Vref) is calculated from the changes in the capacitance of the clothes, and the artifact is removed from the bioelectric signal which is inputted from the electrode 2. Any artifact that is mixed into the bioelectric signal due to the changing of the capacitance of the clothes can thereby be removed, and measurement precision can be improved.

### [Effects]

The effects of the fifth embodiment will be described below.

(7) A reference signal calculation unit 16 (artifact calculating means) which calculates an artifact superimposed to the bioelectric signal in the electrode 2 from changes in the capacitance calculated by the capacitance measuring unit 10, and a subtraction unit 18 (artifact removing means) which subtracts the artifact calculated from the bioelectric signal outputted from the signal separating unit 9, are provided.

Therefore, it is possible to remove any artifact that is mixed into the bioelectric signal due to changes in the capacitance of the clothes, and the measurement precision can be improved.

### [Embodiment 6]

### [Bioelectric signal measuring circuit]

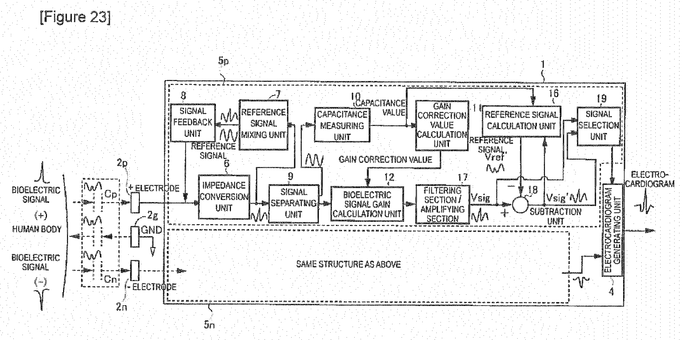

Figure 23 is a control block view of a bioelectric signal measuring circuit 1. The bioelectric signal measuring circuit 1 of the sixth embodiment differs from the bioelectric signal measuring circuit 1 of the fifth embodiment in that a signal selection unit 19 is provided. In the sixth embodiment, the configurations that differ from the first embodiment and the fifth embodiment will be mainly explained, while the configurations that are the same as the first embodiment or the fifth embodiment are given the same codes, and the descriptions thereof are omitted.

### <Signal selection unit>

The signal selection unit 19 is a software installed to a microprocessor. The signal selection unit 19 inputs the bioelectric signal Vsig before being subtraction-corrected and the bioelectric signal Vsig' after being subtraction-corrected in the subtraction unit 18, and selects one or the other of the signals to be outputted to the electrocardiogram generating unit 4.

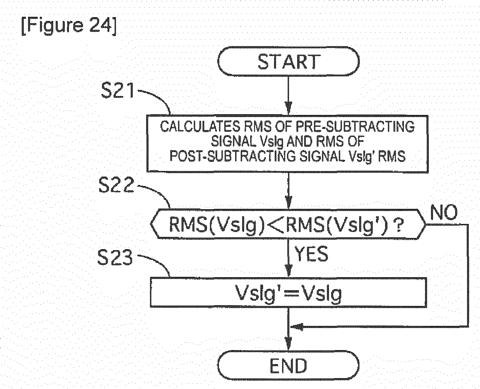

Figure 24 is a flowchart illustrating the flow of the steps of the signal selection unit 19.

In step S11, the root mean square (RMS (Vsig)) of the bioelectric signal Vsig before being subtraction-corrected and the root mean square (RMS (Vsig')) of the bioelectric signal Vsig' after being subtraction-corrected in the subtraction unit 18 are calculated. The root mean square of the bioelectric signal which is received for either a predetermined duration or a predetermined number of times is calculated.

In step S 12, whether or not RMS (Vsig') is larger than RMS (Vsig) is determined, and if RMS (Vsig') is larger, the steps proceeds to step S13, and if RMS (Vsig') is smaller, the steps end.

In step S13, Vsig is substituted as Vsig' and the steps end. That is, the bioelectric signal Vsig before being subtraction-corrected is inputted to the difference means 4.

### [Effects]

RMS (Vsig) and RMS (Vsig') show the variation of the bioelectric signal Vsig before being subtraction-corrected and the variation of the bioelectric signal Vsig' after being subtraction-corrected, respectively. Variation of the bioelectric signal is caused by artifacts mixed into the bioelectric signal due to changes in the capacitance of the clothes, mainly when the occupant moves.

RMS (Vsig') being greater than RMS (Vsig) indicates that movements of the occupant are small, and that almost no artifacts are mixed into the bioelectric signal Vsig before being subtraction-corrected. Alternatively, the above indicates that the set precision of the reference signal Vref is low, in fact causing more artifacts to be mixed into the bioelectric signal by the subtraction-correction, and thereby reducing precision. On the other hand, RMS (Vsig') being equal to or less than RMS (Vsig) indicates that the artifact is reduced by the subtraction-correction and the bioelectric signal precision is improved.

Thus, the sixth embodiment is configured so that the variation of the voltage of the bioelectric signal Vsig' after the subtraction-correction of the reference signal Vref(artifact) by the subtraction unit, and the variation of the voltage of the bioelectric signal Vsig before the subtraction-correction of the reference signal Vref are compared, and the bioelectric signal with the smaller variation is selected in the signal selection unit 19.

Accordingly, artifacts being mixed into the bioelectric signal can be reduced, and the measurement precision can be improved.

### [Effects]

The effects of the sixth embodiment will be described below.

(8) A signal selection unit 19 which compares the variation of the voltage of the bioelectric signal after the subtraction-correcting of artifacts by the subtraction unit 18 (artifact removing means), and the variation of the voltage of the bioelectric signal before the subtraction-correcting of the artifacts, and selects the bioelectric signal with the smaller variation is provided.

Therefore, artifacts being mixed into the bioelectric signal can be reduced, and the measurement precision can be improved.

### [Embodiment 7]

### [Circuit for measuring bioelectric signal]

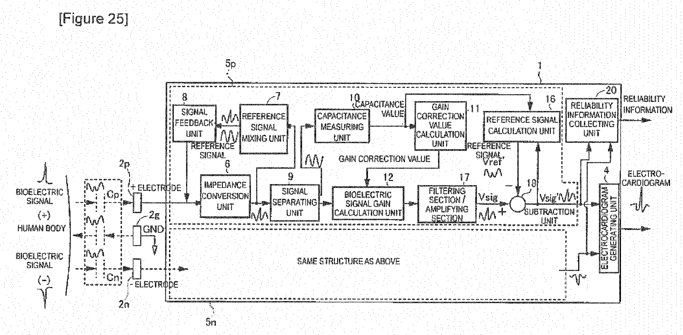

Figure 25 is a control block view of a bioelectric signal measuring circuit 1. The bioelectric signal measuring circuit 1 of the seventh embodiment differs from the bioelectric signal measuring circuit 1 of the sixth embodiment in that a reliability information collecting unit 20 is provided. In the seventh embodiment, the configurations that differ from the first embodiment and the sixth embodiment will be mainly explained, while the configurations that are the same as the first embodiment or the sixth embodiment are given the same codes, and the descriptions thereof are omitted.

### <Reliability information collecting unit>

The reliability information collecting unit 20 collects the variation information of the bioelectric signal Vsig after being subtraction-corrected in the subtraction unit 18 for each electrode. The variation of the bioelectric signal Vsig can be obtained by the root mean square RMS (Vsig) of the bioelectric signal Vsig.

### [Effects]

Variation of the bioelectric signal is caused by artifacts mixed into the bioelectric signal due to changes in the capacitance of the clothes, mainly when the occupant moves. After being subtraction-corrected in the subtraction unit 18, since the artifacts are removed due to the changes in the capacitance, the variation of the bioelectric signal should be small. However, there are cases in which the variation of the bioelectric signal after being subtraction-corrected is increased, due to factors such as the set precision of the reference signal Vref being low.

In other words, an electrocardiogram generated from a portion of the bioelectric signal in which the variation is large has a low precision; therefore, the precision of the electrocardiogram can be determined from the variation of the bioelectric signal. By collecting the above electrocardiogram precisions in the reliability information collecting unit 20, sorting of the electrocardiograms is made possible in the subsequent steps. For example, if the steps requires a high-precision electrocardiogram, only the electrocardiograms having high precision should be selected and used, and if the steps requires a large amount of data even if the precision is somewhat low, electrocardiograms having relatively lower precision may be used as well.

### [Effects]

The effects of the seventh embodiment will be described below.

(9) A reliability information collecting unit 20 for collecting information of the variation of the voltage of the bioelectric signal after the subtraction of the artifacts by the subtraction unit 18 (artifact removing means) of each electrode 2 is provided.

Therefore, information regarding the reliability of the electrocardiogram can be appended along with the electrocardiogram and fed to the subsequent steps.

### [Embodiment 8]

### [Circuit for measuring bioelectric signal]

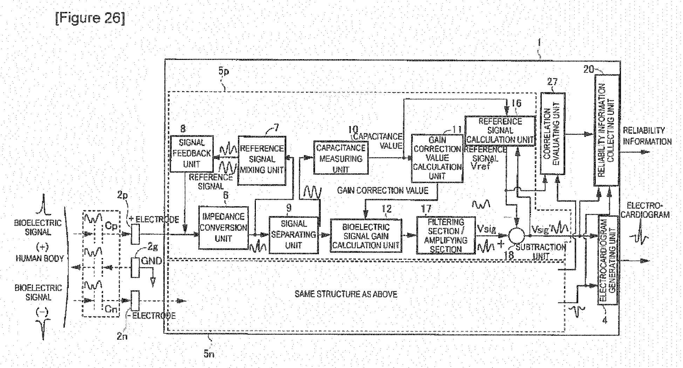

Figure 26 is a control block view of a bioelectric signal measuring circuit 1. The bioelectric signal measuring circuit 1 of the eighth embodiment differs from the bioelectric signal measuring circuit 1 of the seventh embodiment in that a correlation evaluating unit 27 is provided. In the eighth embodiment, the configurations that differ from the first embodiment and the seventh embodiment will be mainly explained, while the configurations that are the same as the first embodiment or the seventh embodiment are given the same codes, and the descriptions thereof are omitted.

### <Correlation evaluating unit>

The correlation evaluating unit 27 calculates the correlation between the reference signals Vref of the electrodes 2. The calculated correlation information is collected in the reliability information collecting unit 20. The correlation between the reference signals Vref can be obtained using a correlation function, or the like.

### [Effects]

In the electrocardiogram generating unit 4, an electrocardiogram is obtained by taking the difference between the positive side and negative side bioelectric signals Vsig'. If the correlation between the positive side reference signal Vref and the negative side reference signal Vref is high, the electrocardiogram error after the differential steps will be small and a high-precision electrocardiogram can be generated. On the other hand, if the correlation between the positive side reference signal Vref and the negative side reference signal Vref is low (in particular, when there is an inverse correlation), artifacts will appear emphasized in the electrocardiogram after the differential steps, and the precision of the electrocardiogram will be low.

In the reliability information collecting unit 20, the reliability of the electrocardiogram according to the correlation of the reference signals Vref can also be collected.

### [Effects]

The effects of the eighth embodiment will be described below.

(10) A correlation evaluating unit 27 for calculating the correlation of the reference signals Vref (artifacts) calculated in the reference signal calculation unit 16 provided to correspond with a plurality of electrodes 2 is provided.

Therefore, the reliability of the electrocardiogram can be calculated from the reference signal Vref, and information regarding the reliability of the electrocardiogram can be appended along with the electrocardiogram and fed to the subsequent steps.

### [Embodiment 9]

### [Circuit for measuring bioelectric

The bioelectric signal measuring circuit of the ninth embodiment differs from the bioelectric signal measuring circuit 1 of the first embodiment in that an electrode selecting unit 26 and a bioelectric signal switching unit 23 are provided. Additionally, the two embodiments differ in that, in the first embodiment, two electrodes, namely, the positive electrode 2p and the negative electrode 2n, are provided in addition to the ground 2g, as the electrodes 2, but in the ninth embodiment, two or more electrodes are provided. In the ninth embodiment, the configurations that differ from the first embodiment will be mainly explained, while the configurations that are the same as the first embodiment are given the same codes, and the descriptions thereof are omitted.

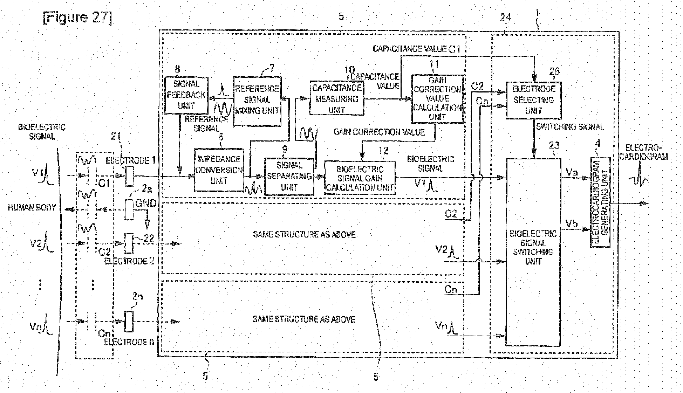

Figure 27 is a control block view of a bioelectric signal measuring circuit 1. The bioelectric signal measuring circuit inputs a bioelectric signal of a human body from electrodes 21, 22, ..., 2n, and outputs the same as an electrocardiogram.

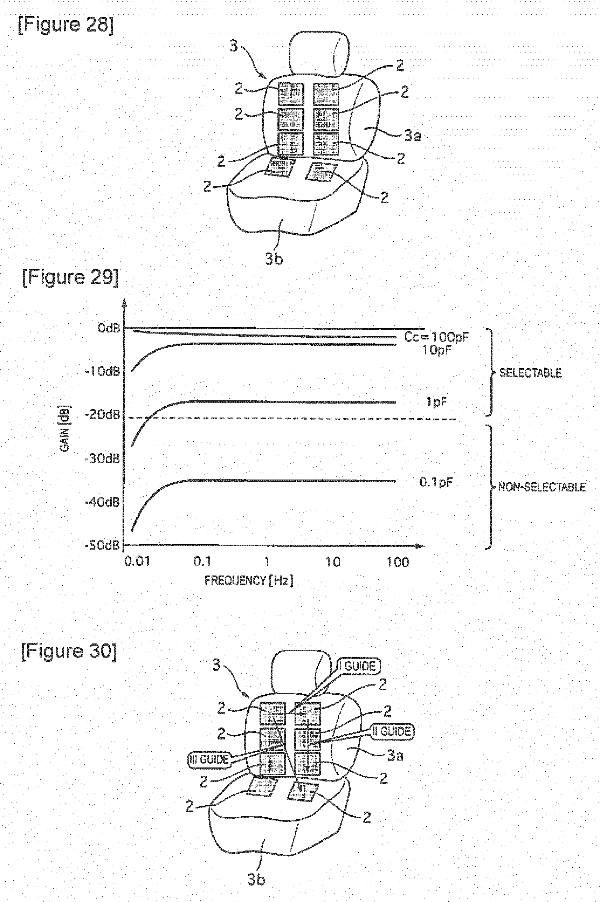

Figure 28 is a schematic view of a seat 3 of a vehicle. The electrode 2 is disposed on the surface of the seat back 3a having insulating properties, and in the seat cushion 3b of the seat 3. A bioelectric signal can thereby be measured simply by an occupant sitting in the seat 3. The electrodes 2 are made of materials having conductive properties, such as metal materials including gold, silver, copper or nichrome, carbon-based materials such as carbon or graphite, particulate materials made of semiconductors of metals and metal oxides, conductive polymer materials such as acetylene-based, 5-membered heterocyclic, phenylene-based or aniline-based compounds, or the like.

The bioelectric signal measuring circuit comprises a gain correction unit 5 which measures the bioelectric signal emitted from the human body and the capacitance of the clothes worn by the occupant, as well as steps and outputs the inputted bioelectric signal, and a bioelectric signal measuring unit 24 which selects and measures the bioelectric signal outputted from each gain correction unit 5.

### [Capacitance measuring unit]

The gain correction unit 5 is provided in accordance with each electrode 21, 22, ..., 2n, but since the configurations are identical, an explanation thereof will be given below without distinction.

A gain correction unit 5 comprises an impedance conversion unit 6, a reference signal mixing unit 7, a signal feedback unit 8, a signal separating unit 9, a capacitance measuring unit 10, a gain correction value calculation unit 11, and a bioelectric signal gain correction unit 12. The configuration of each unit is the same as the first embodiment.

### [Bioelectric signal measuring unit]

The bioelectric signal measuring unit 24 comprises an electrocardiogram generating unit 4 for generating an electrocardiogram from a bioelectric signal, an electrode selecting unit 26 for selecting the bioelectric signal used in the electrocardiogram generating unit 4, and a bioelectric signal switching unit 23 for switching the circuit according to the selected bioelectric signal.

### <Electrode selecting unit>

The electrode selecting unit 26 calculates the output gain of the impedance conversion unit 6 from the capacitance of the clothes calculated by the capacitance measuring unit 10 provided to correspond with each electrode 2. Figure 7 is a map illustrating the output gain of an impedance conversion unit 6 in relation to the capacitance of the clothes. The output gain is represented as the ratio of the signal intensity outputted from the impedance conversion unit 6 in relation to the signal intensity of the bioelectric signal emitted from the human body.

The electrode selecting unit 26 selects the bioelectric signal with the highest output gain and the bioelectric signal with the second highest output gain, from the bioelectric signals having a greater output gain than a predetermined value.

Figure 29 is a graph illustrating the frequency output gain characteristic per capacitance of the clothes. As illustrated in Figure 29, the output gain increases as the capacitance increases. In the electrode selecting unit 26, a selection is made from the electrodes 2 to which has been inputted a bioelectric signal having a greater output gain than a predetermined value (for example, greater than -20 [dB]). Here, the magnitude determination of an output gain may be carried out with an output gain within the range of the frequency band 10-40 [Hz] of the R wave of an electrocardiogram.

### <Bioelectric signal switching unit>

The bioelectric signal switching unit 23 switches the circuit so that a bioelectric signal outputted from the bioelectric signal gain correction unit 12 corresponding to the bioelectric signal selected by the electrode selecting unit 26 is inputted to the electrocardiogram generating unit 4.

### <Electrocardiogram generating unit>

The electrocardiogram generating unit 4 inputs the bioelectric signal outputted from the bioelectric signal gain correction unit 12 corresponding to the selected electrode 2. A filtering step and an amplification step are carried out on the inputted bioelectric signals, and an electrocardiogram is obtained by taking the difference between the positive side signal and the negative side signal after the steps are carried out.

### [Effects]

Clothes having capacitance (C1, C2, ..., Cn) are interposed between the electrode 2 and the human body, since the occupant sits in the seat 3 wearing clothes. The capacitance of the clothes in contact with each electrode 2 differs according to the seating posture of the occupant.

If the capacitance of the clothes is small, the gain of the bioelectric signal outputted from the impedance conversion unit 6 is decreased in relation to the bioelectric signal emitted from the human body (biological composition). If the gain becomes too small, there is the risk that the measurement precision of the bioelectric signal deteriorates.

Therefore, in the ninth embodiment, the capacitance of the clothes in contact with each electrode 2 is calculated, and the output gain of the impedance conversion unit 6 is calculated from the calculated capacitance. Additionally, a bioelectric signal having an output gain greater than a predetermined value is configured to be detected.

Accordingly, since a bioelectric signal with a high output gain can be measured, the measurement precision can be improved.

In addition, in the ninth embodiment, the bioelectric signal with the highest output gain and the bioelectric signal with the second highest output gain, from the bioelectric signals having a greater output gain than a predetermined value, are measured.

Accordingly, since a bioelectric signal with an even higher output gain can be measured, the measurement precision can be improved.

### [Effects]

The effects of the ninth embodiment will be described below.
(11) A plurality of electrodes 2 (input means) for inputting bioelectric signals emitted from a biological composition, and a bioelectric signal measuring unit 24 (bioelectric signal measuring means) which calculates the gain of the bioelectric signal outputted from the impedance conversion unit 6 corresponding to the bioelectric signal emitted by the biological composition from the capacitance calculated by the capacitance measuring unit 10, for measuring bioelectric signals having a gain greater than a predetermined value, are provided.
   Therefore, since a bioelectric signal with a high output gain can be measured, the measurement precision can be improved.
(12) The bioelectric signal measuring unit 24 measures the bioelectric signal with the highest gain and the bioelectric signal with the second highest gain, from the bioelectric signals having a greater gain than a predetermined value.
   Therefore, since a bioelectric signal with an even higher output gain can be measured, the measurement precision can be improved.

### [Embodiment 10]

In the ninth embodiment, in the bioelectric signal measuring unit 24, the bioelectric signal with the highest gain and the bioelectric signal with the second highest gain are measured, from among the bioelectric signals having a greater gain than a predetermined value. In the tenth embodiment, the selection method of the bioelectric signal is different. Since the configurations other than for the selection method of the bioelectric signal are identical to the ninth embodiment, the explanations thereof are omitted.

In the bioelectric signal measuring unit 24, the two bioelectric signals having the smallest gain difference from among the bioelectric signals having a gain greater than a predetermined value are measured.

Accordingly, the error when performing a differential steps in the electrocardiogram generating unit 4 can be reduced, and the measurement precision can be improved.

### [Effects]

The effects of the tenth embodiment will be described below.

(13) The bioelectric signal measuring unit 24 is configured so that the two bioelectric signals having the smallest gain difference from among the bioelectric signals having a gain greater than a predetermined value are measured.

Therefore, the error when performing the differential steps can be reduced, and the measurement precision can be improved.

### [Example 11]

In the ninth embodiment, in the bioelectric signal measuring unit 24, the bioelectric signal with the highest gain and the bioelectric signal with the second highest gain are measured, from among the bioelectric signals having a greater gain than a predetermined value. In the eleventh embodiment, the selection method of the bioelectric signal is different. Since the configurations other than for the selection method of the bioelectric signal are identical to the ninth embodiment, the explanations thereof are omitted.

In the bioelectric signal measuring unit 24, the measuring of the bioelectric signal is carried out in accordance with the first lead, the second lead, and the third lead of the limb lead, from among the bioelectric signals having a gain greater than a predetermined value. A limb lead is a method for measuring the bioelectric signal emitted from the heart. The combination of the electrodes are: the + electrode is attached to the left hand, and the - electrode is attached to the right hand, for the first lead; the + electrode is attached to the left foot, and the - electrode is attached to the right hand, for the second lead, and; the + electrode is attached to the left foot, and the - electrode is attached to the left hand, for the third lead.

Figure 30 is a view illustrating an example of a limb lead. In the eleventh embodiment, since the electrodes are provided in the seat 3, the electrodes cannot be directly attached to the left hand, the right hand, or the left foot, although a limb lead can be realized by using the electrode in the closest position to each location. Among the leads of a limb lead, the waveform of the second lead is drawn most clearly; subsequently the first lead, and lastly the third lead are drawn with clarity decreasing in that order.

Therefore, first, the bioelectric signal corresponding to the second lead is configured to be selected and measured. When the measuring of the bioelectric signal according to the second lead cannot be carried out, for reasons such as the gain of the bioelectric signal attempted to be selected being smaller than the predetermined value, the bioelectric signal corresponding to the first lead is selected and measured. If the measuring of the bioelectric signal according to the first lead cannot be carried out as well, the bioelectric signal corresponding to the third lead is selected and measured.

Accordingly, by utilizing the bioelectric signal whose waveform is drawn clearly, the measurement precision can be improved.

### [Effects]

The effects of the eleventh embodiment will be described below.

(14) The bioelectric signal measuring unit 24 is configured so that, among the bioelectric signals having a gain greater than a predetermined value: the bioelectric signal inputted from the electrode 2 closest to the left foot of a biological composition and the bioelectric signal inputted from the electrode 2 closest to the right hand are measured (second lead); if the measuring of the bioelectric signal according to the second lead cannot be carried out, the bioelectric signal inputted from the electrode 2 closest to the left hand of the biological composition and the bioelectric signal inputted from the electrode 2 closest to the right hand are measured (first lead), and; if the measuring of the bioelectric signal according to the first lead cannot be carried out, the bioelectric signal inputted from the electrode 2 closest to the left foot of the biological composition and the bioelectric signal inputted from the electrode 2 closest to the left hand are measured (third lead).

Therefore, by utilizing the bioelectric signal whose waveform is drawn clearly, the measurement precision can be improved.

### [embodiment 12]

In the ninth embodiment, in the bioelectric signal measuring unit 24, the bioelectric signal with the highest gain and the bioelectric signal with the second highest gain are measured, from among the bioelectric signals having a greater gain than a predetermined value. In the twelfth embodiment, the selection method of the bioelectric signal is different. Since the configurations other than for the selection method of the bioelectric signal are identical to the ninth embodiment, the explanations thereof are omitted.

In the bioelectric signal measuring unit 24, the bioelectric signal with the highest SN ratio and the bioelectric signal with the second highest SN ratio are measured, from among the bioelectric signals having a greater gain than a predetermined value. Accordingly, noise in the bioelectric signal to be measured is small, and the measurement precision can be improved.

### [Effects]

The effects of the twelfth embodiment will be described below.

(15) The bioelectric signal measuring unit 24 is configured so that the bioelectric signal with the highest SN ratio and the bioelectric signal with the second highest SN ratio are measured, from among the bioelectric signals having a greater gain than a predetermined value.

Therefore, noise in the bioelectric signal to be measured can be reduced, and the measurement precision can be improved.

### [Embodiment 13]

In embodiments 9 through 12, in the bioelectric signal measuring unit 24, the bioelectric signals to be measured are selected by respective selection methods, from among the bioelectric signals having a gain greater than a predetermined value. The thirteenth embodiment is configured so that when the gain of the selected bioelectric signal, selected by one of the selection methods of embodiments 9 through 12, falls below the predetermined value, another bioelectric signal is selected midway. Since the configurations other than for the selection method of the bioelectric signal are identical to the ninth embodiment, the explanations thereof are omitted.

In the thirteenth embodiment, whether or not to select another bioelectric signal is determined not by directly using the gain changes; rather, the determination is carried out by using the changes of the capacitance between the biological composition and the electrode 2 to which the bioelectric signal is inputted.

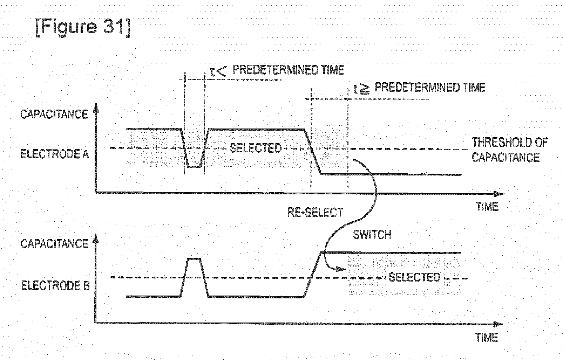

Figure 31 is a graph illustrating an example of the changing of the capacitance between the electrode 2 and a biological composition. Changes in the capacitance between a biological composition and an electrode (electrode A), as well as another electrode (electrode B), are illustrated in Figure 31 as an example. Here, let us first assume that the bioelectric signal inputted to electrode A is selected and measured. As long as the duration t in which the capacitance between the electrode A and the biological composition it equal to or less than the predetermined value is shorter than a predetermined duration, the bioelectric signal inputted to the electrode A remains selected. The capacitance becoming equal to or less than a predetermined value indicates that the gain of the bioelectric signal has become equal to or less than the predetermined value described in the ninth embodiment.

If the duration t in which the capacitance between the electrode A and the biological composition it equal to or less than the predetermined value exceeds a predetermined duration, a bioelectric signal inputted to another electrode B having a capacitance between the biological composition that is greater than the predetermined value, is selected. The selection of a new bioelectric signal may be done based on the respective selection methods described in embodiments 9 through 12.

Accordingly, even if the gain is temporarily decreased, the bioelectric signal is not reselected, and the measuring of the bioelectric signal can be stably carried out. On the other hand, when the gain is steadily reduced, the measurement precision can be improved by measuring a different bioelectric signal.

### [Effects]

The effects of the thirteenth embodiment will be described below.

(16) The bioelectric signal measuring unit 24 is configured so that if the duration in which the capacitance between the biological composition and the electrode 2, to which the bioelectric signal used for measurement is inputted, stays below a predetermined value exceeds a predetermined duration, a bioelectric signal inputted from another electrode 2 is measured.

Therefore, even if the gain is temporarily reduced, the bioelectric signal is not reselected, and the measuring of the bioelectric signal can be stably carried out. On the other hand, when the gain is steadily reduced, the measurement precision can be improved by measuring a different bioelectric signal.

### [Embodiment 14]

The thirteenth embodiment is configured so that when the gain of the selected bioelectric signal becomes equal to or less than the predetermined value, another bioelectric signal is selected midway. The fourteenth embodiment is configured so that a bioelectric signal whose output gain change amount is smaller than a predetermined value is selected when selecting another bioelectric signal. Since the configurations other than for the selection method of the bioelectric signal are identical to the ninth embodiment, the explanations thereof are omitted.

In the fourteenth embodiment, whether or not to select another bioelectric signal is determined not by directly using the gain change amount; rather, the determination is carried out by using the change amount of the capacitance between the biological composition and the electrode 2 to which the bioelectric signal is inputted. That is, the history of capacitance change of the clothes in contact with each electrode 2 is recorded, and the bioelectric signal inputted from the electrode 2 whose capacitance change amount is smaller than a predetermined value is selected.

Accordingly, the possibility of reselecting is decreased, and the measuring of the bioelectric signal can be stably carried out.

### [Effects]

The effects of the fourteenth embodiment will be described below.

(17) The bioelectric signal measuring unit 24 is configured so that the history of capacitance change between the biological composition and each electrode 2 is recorded, and the bioelectric signal inputted from another electrode 2 whose capacitance change amount is smaller than a predetermined value is measured.

Therefore, the possibility of reselecting is decreased, and the measuring of the bioelectric signal can be stably carried out.

### [Embodiment 15]

In the fourteenth embodiment, the history of capacitance change is recorded. In the fifteenth embodiment, the behavior of the occupant (or the clothes worn by the occupant) is inferred, and the capacitance change is inferred from the previously behavior.

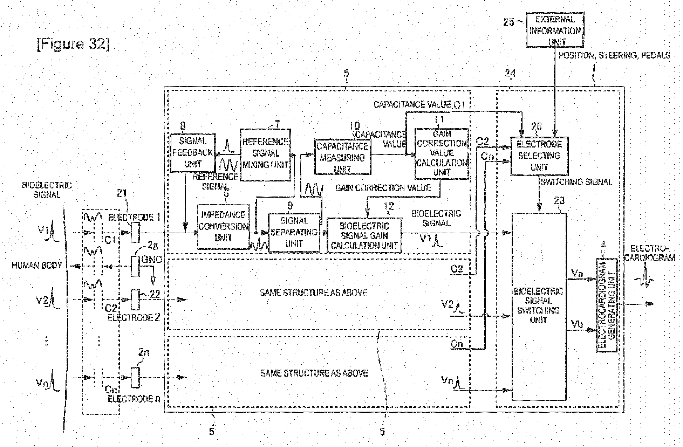

Figure 32 is a control block view of a bioelectric signal measuring circuit 1. As illustrated in Figure 11, an external information unit 25 is connected to the electrode selecting unit 26. Information such as accelerator pedal operating information, brake pedal operating information, and steering wheel steering information by CAN communication, and information from a vehicle behavior control apparatus, a navigation system, or the like are inputted from the external information unit 25.

Inferring the behavior of the occupant (clothes) is carried out by, for example, inferring the behavior of the vehicle based on an accelerator pedal operation, a brake pedal operation, or information from a vehicle behavior control apparatus, and inferring the behavior of the occupant (clothes) accompanying the behavior change of the vehicle. Alternatively, the behavior of the occupant (clothes) accompanying the behavior change of the vehicle is inferred by using information form the navigation system and inferring the vehicle behavior.

Accordingly, the possibility of reselecting is decreased, and the measuring of the bioelectric signal can be stably carried out.

### [Effects]

The effects of the fifteenth embodiment will be described below.
(18) An external information unit 25 (behavior inferring means) for inferring the behavior of the occupant (biological composition) is provided, wherein the bioelectric signal measuring unit 24 infers the capacitance change from the inferred occupant behavior, and measures the bioelectric signal inputted from another electrode 2 whose inferred capacitance change amount is smaller than a predetermined value.
   Therefore, the possibility of reselecting is decreased, and the measuring of the bioelectric signal can be stably carried out.
(19) The external information unit 25 is configured to infer the occupant behavior using information from the navigation system.
   Therefore, the possibility of reselecting is decreased, and the measuring of the bioelectric signal can be stably carried out.
(20) The external information unit 25 is configured to infer the occupant behavior using operational information for operating the vehicle.
   Therefore, the possibility of reselecting is decreased, and the measuring of the bioelectric signal can be stably carried out.

### (Other embodiments)

The present invention is not limited to the configurations described in the above embodiments, and other configurations are also possible.

For example, the signal intensity calculation of the reference signal in step S1 of the first embodiment is implemented as a software, but may be configured from an analog circuit capable of carrying out similar steps using a detection circuit, etc.

In addition, in the first embodiment, the capacitance measuring unit 10 is configured from a software installed to a microprocessor, but may be configured from an analog circuit.

In the second embodiment, the reference signal intensity changing unit 14 is configured from a switch circuit, but may be configured so that the signal intensity of the inputted reference signal itself is changed.

Additionally, the flattening function unit 7a of the third embodiment may be applied to the reference signal mixing unit 7 of the second embodiment. In that case, a capacitor may be provided, connected in series to the resistors R11 and R1.

For example, in the fourth embodiment, the switch SW of the resonance suppressing unit 15 is configured to switch between a circuit which passes through a capacitor Cin and a circuit which does not pass through a capacitor Cin. However, the switch may be configured to switch in two or more stages, by providing a plurality of capacitors Cin with different capacities.

## Claims

1. A bioelectric signal measuring circuit, comprising:
an input means for inputting a bioelectric signal emitted from a biological composition;
an impedance converting means for converting the impedance of a bioelectric signal inputted to the input means;
a reference signal mixing means which mixes an output signal of the impedance converting means and a reference signal for calculating the capacitance between the biological composition and the input means;
a signal feedback means for feeding back the output signal of the reference signal mixing means to the impedance converting means;
a signal separating means for separating the bioelectric signal and the reference signal from the output signal of the impedance converting means;
a gain correction value calculating means for calculating a gain correction value of the bioelectric signal, based on a capacitance measuring means for calculating the capacitance from the intensity of the reference signal inputted from the signal separating means; and
a bioelectric signal gain correction means for carrying out a gain correction of the bioelectric signal based on the gain correction value.

2. The bioelectric signal measuring circuit according to claim 1, comprising:
a reference signal intensity setting value calculation means for setting the intensity of the reference signal inputted to the reference signal mixing means based on the intensity of the reference signal inputted from the signal separating means; and
a reference signal intensity changing means for changing the intensity of the reference signal inputted to the reference signal mixing means in accordance with the intensity of the reference signal set by the reference signal intensity setting value calculation means; wherein
the capacitance measuring means calculates the capacitance from the intensity of the reference signal inputted from the signal separating means and the intensity of the reference signal set by the reference signal intensity setting value calculation means.

3. The bioelectric signal measuring circuit according to claim 2, wherein
the reference signal intensity changing means carries out the changing of the intensity of the reference signal inputted to the reference signal mixing means by changing the constant of an electrical circuit in the reference signal mixing means.

4. The bioelectric signal measuring circuit according to any one of claim 1 to claim 3,
wherein
the reference signal mixing means comprises a flattening function unit set to flatten the gain characteristic of the reference signal outputted from the impedance converting means in relation to the frequency change of the reference signal.

5. The bioelectric signal measuring circuit according to any one of claim 1 to claim 4,
wherein
the signal feedback means comprises two resistors connected in series in a circuit connecting the input terminal of the impedance converting means and a ground, and
a capacitor and a resistor connected in series in a circuit connecting between the two resistors and the output terminal of the reference signal mixing means; and
a capacitor is provided in series between the input means and the input terminal of the impedance converting means, when the capacitance calculated by the capacitance measuring means is greater than a predetermined value.

6. The bioelectric signal measuring circuit according to any one of claim 1 to claim 5,
wherein
an artifact calculating means for calculating an artifact superimposed to the bioelectric signal in the input means based on the changes of the capacitance calculated by the capacitance measuring means; and
an artifact removing means for subtracting the calculated artifact from the bioelectric signal outputted from the signal separating means.

7. The bioelectric signal measuring circuit according to claim 6, wherein
a signal selection means which compares the variation of the bioelectric signal voltage after the artifact is subtracted by the artifact removing means, and
the variation of the bioelectric signal voltage before the artifact is subtracted, and selects the bioelectric signal having the smaller variation.

8. The bioelectric signal measuring circuit according to claim 6 or claim 7, wherein
a reliability information collecting means for collecting information of the variation of the bioelectric signal voltage, after the artifact is subtracted by the artifact removing means, of each electrode.

9. The bioelectric signal measuring circuit according to any one of claim 6 to claim 8,
wherein
a correlation evaluating means for calculating the correlation of the artifact calculated in the artifact calculating means, provided to correspond with a plurality of input means.

10. The bioelectric signal measuring circuit according to any one of claim 1 to claim 9,
comprising:
a plurality of the input means, wherein
a bioelectric signal measuring means for measuring the bioelectric signal whose gain is higher than a predetermined value, by calculating the gain of the bioelectric signal outputted from the impedance converting means in relation to the bioelectric signal emitted by the biological composition, based on the capacitance calculated by the capacitance measuring means.

11. The bioelectric signal measuring circuit according to claim 10, wherein
the bioelectric signal measuring means measures the bioelectric signal with the highest gain and the bioelectric signal with the second highest gain from among the bioelectric signals having gains higher than a predetermined value.

12. The bioelectric signal measuring circuit according to claim 10, wherein
the bioelectricity measuring means measures the two bioelectric signals having the smallest gain difference from among the bioelectric signals having gains higher than a predetermined value.

13. The bioelectric signal measuring circuit according to claim 10, wherein
the bioelectricity measuring means, from among the bioelectric signals having gains higher than a predetermined value:
measures, of the biological composition, the bioelectric signal inputted from the input means closest to the left foot and the bioelectric signal inputted from the input means closest to the right hand (second lead);
measures, of the biological composition, the bioelectric signal inputted from the input means closest to the left hand and the bioelectric signal inputted from the input means closest to the right hand (first lead), when the measuring of the bioelectric signal according to the second lead cannot be carried out; and
measures, of the biological composition, the bioelectric signal inputted from the input means closest to the left foot and the bioelectric signal inputted from the input means closest to the left hand (third lead), when the measuring of the bioelectric signal according to the first lead cannot be carried out.

14. The bioelectric signal measuring circuit according to claim 10, wherein
the bioelectric signal measuring means, from among the bioelectric signals having gains higher than a predetermined value, measures the bioelectric signal having the highest SN ratio and the bioelectric signal having the second highest SN ratio.

15. The bioelectric signal measuring circuit according to any one of claim 10 to claim 14,
wherein
the bioelectricity measuring means measures the bioelectric signal inputted from another of the input means, when the duration in which the capacitance, between the input means for inputting the bioelectric signal used for measuring and the biological composition, becomes equal to or less than a predetermined value exceeds a predetermined duration.

16. The bioelectric signal measuring circuit according to claim 15, wherein
the bioelectricity measuring means records a history of the capacitance changes between the biological composition and each of the input means, and measures the bioelectric signal inputted from another of the input means whose change amount of the capacitance is smaller than a predetermined value.

17. The bioelectric signal measuring circuit according to claim 15, wherein
a behavior inferring means for inferring the behavior of the biological composition, wherein
the bioelectricity measuring means infers the capacitance change from the inferred behavior of the biological composition, and
measures the bioelectric signal inputted from the other input means whose change amount of the inferred capacitance is smaller than a predetermined value.

18. The bioelectric signal measuring circuit according to claim 17, wherein
the behavior inferring means infers the behavior of the biological composition using information from a navigation system.

19. The bioelectric signal measuring circuit according to claim 17, wherein
the behavior inferring means infers the behavior of the biological composition using operational information for operating a vehicle.

20. The bioelectric signal measuring circuit according to any one of claim 1 to claim 19, wherein
the input means is disposed in a seat of a vehicle.
